# EUROPÄISCHE PATENTSCHRIFT

(11) **EP 1 755 440 B1**
(45) Veröffentlichungstag und Bekanntmachung des Hinweises auf die Patenterteilung: **25.08.2010**
(21) Anmeldenummer: 05750185.0
(22) Anmeldetag: 01.06.2005
(51) Int. Cl.: A61B 1/32, A61B 17/34, A61M 29/00

(54) **VORRICHTUNG ZUM AUFWEITEN VON ORGAN- ODER KÖRPERÖFFNUNGEN**
DEVICE FOR EXPANDING ORGAN OR BODY OPENINGS
DISPOSITIF PERMETTANT D'ELARGIR DES OUVERTURES DANS UN ORGANE OU UN ORGANISME

(30) Priorität: 03.06.2004 DE 202004008827 U
(43) Veröffentlichungstag der Anmeldung: 28.02.2007
(73) Patentinhaber: Polydiagnost GmbH, 85276 Pfaffenhofen (DE)
(72) Erfinder: SCHAAF, Hansgeorg, 85293 Reichertshausen (DE)
(74) Vertreter: Eisenführ, Speiser & Partner
(86) Internationale Anmeldenummer: PCT/EP2005/005887
(87) Internationale Veröffentlichungsnummer: WO 2005/117687

(56) Entgegenhaltungen:
- EP-A- 0 450 221
- EP-A- 1 293 228
- US-A- 5 417 665
- US-A1- 2002 103 446

## Beschreibung

Die Erfindung betrifft eine Vorrichtung zum Aufweiten von Organ- oder Körperöffnungen mit kleinem Durchmesser, von insbesondere 0,5 mm und geringer.

Es ist bekannt, mit Hilfe von konisch verlaufenden Bougierstäben, beispielsweise bei der Stenosenaufdehnung, kleinste Organ- und Körperöffnungen aufzuweiten. Die Aufweitung erfolgt dabei mit Hilfe starrer oder flexibler Dehnsonden unterschiedlicher Dicke, um anschließend ein Instrument oder Endoskop in die aufgeweitete Organ- bzw. Körperöffnung einzubringen.

Aus DE 100 65 604 C2 ist ein stabförmiges Instrument mit starrem konischen Hohlkörper zur Tracheal-Bougierung bekannt.

Aus DE 84 07 894 U1 ist ein Dilatator mit konischem Hohlkörper zum Aufweiten von Einstichkanälen der Niere bekannt.

Aus DE 202 09 595 ist ebenfalls ein Dilatator zum Aufweiten von Organ- oder Körperöffnungen bekannt, bei dem ein distaler Endbereich eines konischen Hohlkörpers verformbar ausgebildet ist und in einem Innenraum eines konischen Hohlkörpers ein Expander eingeschoben werden kann, durch den der distale Endbereich des konischen Hohlkörpers expandiert wird.

Aufgabe der Erfindung ist es, eine Vorrichtung zum Aufweiten von Organ- oder Körperöffnungen zu schaffen, die einen einfachen Aufbau aufweist und mit welcher eine schonende Dilatation der Organ- oder Körperöffnung erreicht wird.

Diese Aufgabe wird erfindungsgemäß durch das Kennzeichen des Patentanspruches 1 gelöst.

Hierzu besitzt die Vorrichtung einen massiven und starren Mandrin, der aus einem Vollkörper gebildet ist, dessen starre distale Mandrinspitze abgerundet ist. An die Mandrinspitze schließt sich ein stabförmiger Mandrinteil an, der im wesentlichen eine kreiszylindrische Außenfläche aufweist. An den stabförmigen Mandrinteil schließt sich ein konischer Mandrinteil an, der einen Konuswinkel von vorzugsweise 22° bis 23° und insbesondere 22°37' aufweist. An den konischen Mandrinteil schließt sich ein Führungsteil mit gleichbleibendem Querschnitt an.

Ferner besitzt die Vorrichtung einen Katheter mit einem konischen distalen Katheterende, das den gleichen Konuswinkel aufweist, wie der konische Mandrinteil oder einen geringeren Konuswinkel. Das Katheterlumen ist dem gleichbleibenden Querschnitt des Führungsteils des Mandrins angepasst.

Der Mandrin kann in das Katheterlumen eingeschoben werden, wobei durch einen Anschlag gewährleistet ist, dass die Mandrinspitze, der sich anschließende stabförmige Mandrinteil und der konische Mandrinteil, welche ein Dilatatorteil des Mandrins bilden, über das distale Katheterende hinaus geschoben werden. Durch einen Anschlag ist gewährleistet, dass die konisch verlaufende Katheterspitze sich an das Konusende mit dem größeren Querschnitt des konischen Mandrinteils anschließt. Hierdurch wird eine konische Dilatatorfläche erzielt, welche sich bis zum kreiszylindrischen Außenumfang des Katheters fortsetzt. Beim Einbringen des Katheters in die Organ- bzw. Körperöffnung wird hierdurch zunächst eine schonende Dilatation der Körperöffnung erreicht, an die sich das Einschieben des distalen, konischen Katheterendes bis zum endgültigen konstanten Außenumfang des Katheters fortsetzt.

Anhand der Figuren wird an Ausführungsbeispielen die Erfindung noch näher erläutert.

Es zeigt
- Fig. 1: in teilweise geschnittener Darstellung eine Seitenansicht eines Ausführungsbeispiels der Erfindung;
- Fig. 2: einen Mandrin, welcher beim Ausführungsbeispiel verwendet wer- den kann;
- Fig. 3: einen Katheter, welcher beim Ausführungsbeispiel der Fig. 1 ver- wendet werden kann;
- Fig. 4: in perspektivischer Darstellung ein weiteres Ausführungsbeispiel für einen Katheter;
- Fig. 5: in perspektivischer Darstellung ein weiteres Ausführungsbeispiel eines zum Katheter der Fig. 4 gehörigen Mandrins;
- Fig. 6: in perspektivischer Darstellung den zusammengebauten Zustand von Katheter der Fig. 4 und Mandrin der Fig. 5;
- Fig. 7: in Draufsicht das Ausführungsbeispiel des Katheters der Fig. 4;
- Fig. 8: in Draufsicht das Ausführungsbeispiel des Mandrins der Fig. 5; und
- Fig. 9: in Draufsicht den Zusammenbau von Katheter der Fig. 4 und 7 sowie von Mandrin der Fig. 5 und 8.

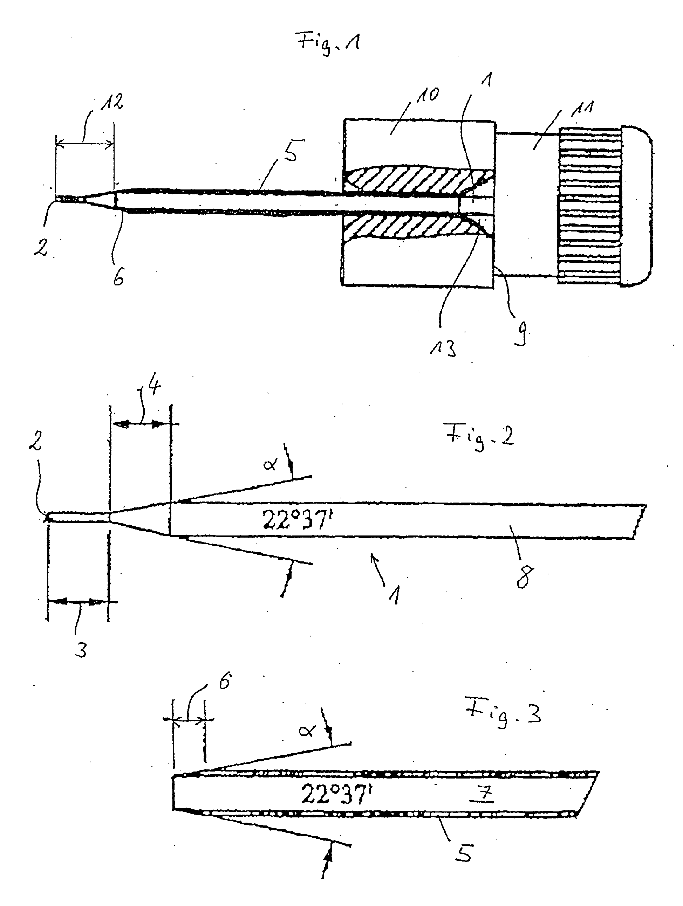

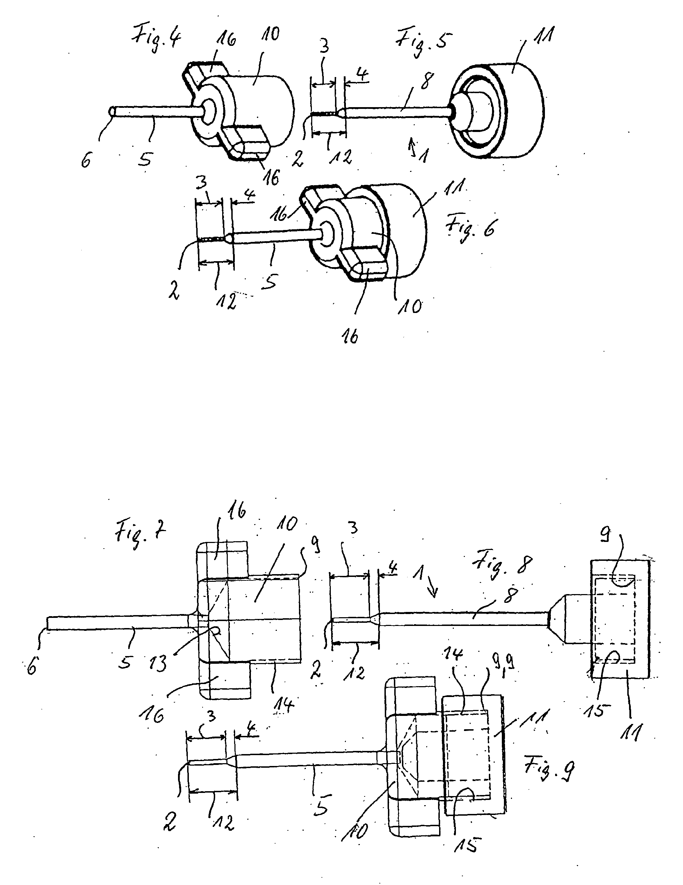

Die in den Figuren dargestellten Ausführungsbeispiele einer Vorrichtung zum Aufweiten von Organ- und Körperöffnungen besitzen einen massiven aus einem Vollmaterial bestehenden starren Mandrin 1 (Fig. 2, 5, 8), der eine abgerundete Mandrinspitze 2 aufweist. An die Mandrinspitze schließt sich ein stabförmiger Mandrinteil 3 an, der eine kreiszylindrische Umfangsfläche aufweist. Hieran schließt sich ein konischer Mandrinteil 4 an, der einen Konuswinkel α, beispielsweise von 22°37' (Fig. 1 bis 3) und 22° (Fig. 4 bis 8) aufweist. An den konischen Mandrinteil 4 schließt sich ein Führungsteil 8 mit gleichbleibendem Querschnitt, insbesondere mit kreiszylindrischem Umfang an.

Die distale Mandrinspitze 2, der stabförmige Mandrinteil 3 und der konische Mandrinteil 4 bilden am distalen Ende des Mandrins 1 ein Dilatatorteil 12, mit welchem die Organ- oder Körperöffnung beim Einschieben aufgeweitet wird.

Der Mandrin 1 kann, wie in den Fig. 1, 6 und 9 dargestellt ist, in ein Katheterlumen 7 eines Katheters 5, von welchem in den Fig. 3, 4 und 7 Ausführungsbeispiele dargestellt sind, eingeschoben werden. Zur Erleichterung des Einschiebens besitzt ein am proximalen Ende des Katheters 5 befestigtes Anschlussstück 10 einen Einschubtrichter 13, welcher das proximale Ende des Katheterlumens 7 umgibt. Das proximale Ende des Mandrins 1 ist an einem Betätigungsteil 11, beispielsweise einem Einschubkopf befestigt. Zwischen dem Anschlussstück 10 und dem Betätigungsteil 11 kann ein Anschlag 9 oder können Anschläge 9 zur Wirkung kommen, durch welchen bzw. welche die Einschubstrecke des Mandrins 1 in das Katheterlumen 7 begrenzt wird.

Diese Begrenzung erfolgt in der Weise, dass das Dilatatorteil 12 des Mandrins 1, welches oben beschrieben wurde, aus dem distalen Katheterende 6 ragt. Dabei ist gewährleistet, dass ein kontinuierlicher Übergang zwischen der konisch sich erweiternden Fläche des Mandrinteils 4 in die konische Fläche des distalen Katheterendes 6 übergeht. Das distale Katheterende 6 weist hierzu ebenfalls eine konische Außenfläche auf, welche den gleichen Konuswinkel aufweist, wie der konische Mandrinteil 4. Der Konuswinkel des Katheterendes 6 kann auch geringfügig geringer sein. Zwischen dem hinteren (proximalen) Ende des konischen Mandrinteils 4 und dem vorderen (distalen) Ende des konischen Katheterendes 6 kann ein geringer Abstand von etwa 0,20 mm vorhanden sein. Der Abstand kann auf Null reduziert werden.

Bei den Ausführungsbeispielen der Fig. 1 bis 3 kann die Gesamtlänge des Katheters 5 einschließlich des Anschlussstückes 10 etwa 25,0 mm betragen. Die Länge des Dilatatorteils 12 am distalen Ende des Mandrins 1 kann 4,0 mm bis 6,0 mm betragen. Der Durchmesser des stabförmigen Mandrinteils 3 beträgt 0,30 mm. Die Länge des stabförmigen Mandrinteils kann 2,0 mm bis 4,0 mm betragen. Der konische Mandrinteil 4 hat eine Länge von etwa 2,0 mm.

Der Außendurchmesser des Katheters beträgt etwa 1,50 mm. Der Innendurchmesser des Katheters beträgt etwa 1,20 mm. Die Länge des konisch geformten Katheterendes 6 beträgt ca. 1,0 mm. Der Außendurchmesser des Mandrins 1 im Bereich des Führungsteils 8 beträgt 1,10 mm.

Die Länge des Anschlussstückes, welches das proximale Endstück des Katheters 5 aufnimmt, beträgt ca. 10,0 mm.

Die Abmessungen des in den Fig. 4 bis 8 dargestellten Ausführungsbeispiels weichen hiervon geringfügig ab. Die Länge des Katheters 5 beträgt etwa 15,0 mm. Die Länge des Dilatatorteils 12 am distalen Ende des Mandrins 1 beträgt etwa 5,0 mm. Der Durchmesser des stabförmigen Mandrinteils 3 beträgt 0,45 mm. Die Länge des stabförmigen Mandrinteils 3 beträgt etwa 4,0 mm. Der konische Mandrinteil 4 hat eine Länge von etwa 1,0 mm. Der Außendurchmesser des Katheters 5 beträgt etwa 1,30 mm und sein Innendurchmesser beträgt etwa 1,22 mm. Die axiale Länge des abgeschrägten distalen Endes des Katheters 5 kann etwa 0,07 mm betragen. Der Durchmesser des Führungsteils 8 des Mandrins 1 beträgt 1,22 mm.

Beim Ausführungsbeispiel der Fig. 4 bis 9 besitzt das Anschlussstück 10 einen topfförmigen Teil, an dessen vorderem Ende zwei diametral abstehende Vorsprünge 16 vorgesehen sind. Hieran schließt sich ein Außengewinde 14 an. Am Boden des topfförmigen Anschlussstückes 10 befindet sich der Einschubtrichter 13, welcher ein erleichtertes Einführen des Mandrins 1 in das Lumen des Katheters 5 gewährleistet.

Das Betätigungsteil 11 ist etwa kappenförmig ausgebildet und besitzt ein Innengewinde 15, mit welchem das Betätigungsteil 11 auf das Außengewinde 14 des Anschlussstückes 10 aufschraubbar ist. Der Kappenboden des Betätigungsteils 11 bildet eine Anschlagfläche für einen am Ende des topfförmigen Anschlussstückes 10 vorgesehenen Anschlag 9, welcher am Rand des topfförmigen Teils gebildet wird. Durch die beiden Anschläge 9, 9 wird die Einschubstrecke des Mandrins 1 in den Katheter 5 begrenzt. Hierdurch ist gewährleistet, dass der Mandrin 1 mit der gewünschten Länge, insbesondere mit dem Dilatatorteil 12 über das distale Katheterende 6 ragt. Der Mandrin 1 ist über ein Sockelteil am Boden des kappenförmigen Betätigungsteils 11 befestigt, wie insbesondere aus den Fig. 8 und 9 zu ersehen ist. Die Wand des topfförmigen Anschlussstückes 10 ist so bemessen, dass sie beim Einschrauben zwischen das Sockelteil und die Innenfläche des Innengewindes 15 passt. Im zusammengebauten Zustand erleichtern die beiden diametralen Vorsprünge 16 die Handhabung der Expansionsvorrichtung.

### Bezugszeichenliste

- 1: Mandrin
- 2: Mandrinspitze
- 3: stabförmiger Mandrinteil
- 4: konischer Mandrinteil
- 5: Katheter
- 6: distales Katheterende
- 7: Katheterlumen
- 8: Führungsteil des Mandrins
- 9: Anschlag
- 10: Anschlussstück
- 11: Betätigungsteil
- 12: Dilatatorteil
- 13: Einschubtrichter
- 14: Außengewinde
- 15: Innengewinde
- 16: Vorsprünge

## Patentansprüche

1. Vorrichtung zum Aufweiten von Organ- oder Körperöffnungen, **gekennzeichnet durch**
- einen massiven und starren Mandrin (1) mit abgerundeter Mandrinspitze (2), einem sich an die Mandrinspitze (2) anschließenden stabförmigen Mandrinteil (3), einem sich an den stabförmigen Mandrinteil (3) anschließenden konischen Mandrinteil (4) und einem an den konischen Mandrinteil (4) sich anschließenden Führungsteil (8) mit gleichbleibendem Querschnitt;
- einen Katheter (5) mit einem konischen, distalen Katheterende (6), das höchstens den gleichen Konuswinkel (α) aufweist wie der konische Mandrinteil (4) und einem dem Querschnitt des Führungsteils (8) angepassten Katheterlumen (7); und
- einen Anschlag (9), der bei aus dem distalen Katheterende (6) ragendem, aus Mandrinspitze, stabförmigen Mandrinteil (3) und konischem Mandrinteil (4) gebildeten Dilatatorteil (12) des Mandrins (1) zwischen dem Mandrin (1) und dem Katheter (5) wirksam ist.

2. Vorrichtung nach Anspruch 1,
**dadurch gekennzeichnet, dass** das distale konische Katheterende (6) sich unmittelbar an das größere Konusende des konischen Mandrinteils (4) anschließt.

3. Vorrichtung nach Anspruch 1 oder 2,
**dadurch gekennzeichnet, dass** das proximale Mandrinende an einem Betätigungsteil (11) und das proximale Katheterende an einem Anschlussstück (10) befestigt sind, wobei der Anschlag (9) zwischen dem Betätigungsteil (11) und dem Anschlussstück (10) gebildet ist.

4. Vorrichtung nach Anspruch 3,
**dadurch gekennzeichnet, dass** am Anschlussstück (10) ein das proximale Ende des Katheterlumens (7) umfassender Einschubtrichter (13) vorgesehen ist.

5. Vorrichtung nach Anspruch 1,
**dadurch gekennzeichnet, dass** der stabförmige Mandrinteil (3) einen Durchmesser von 0,30 mm aufweist.

6. Vorrichtung nach Anspruch 1 oder 5,
**dadurch gekennzeichnet, dass** der stabförmige Mandrinteil (3) eine Länge von 2,0 mm bis 4,0 mm aufweist.

7. Vorrichtung nach Anspruch 1,
**dadurch gekennzeichnet, dass** der konische Mandrinteil (4) eine Länge von 2,0 mm aufweist.

8. Vorrichtung nach Anspruch 1,
**dadurch gekennzeichnet, dass** der Konuswinkel (α) des konischen Mandrinteils mindestens 22° und höchstens 23° beträgt.

9. Vorrichtung nach Anspruch 1,
**dadurch gekennzeichnet, dass** der konische Teil des distalen Katheterendes (6) eine Länge von etwa 1,0 mm hat.

10. Vorrichtung nach Anspruch 1,
**dadurch gekennzeichnet, dass** die Querschnitte des Katheterlumens (7) und des Führungsteils (8) des Mandrins (1) kreiszylindrisch ausgebildet sind.

11. Vorrichtung nach Anspruch 1 oder 10,
**dadurch gekennzeichnet, dass** der Durchmesser des Führungsteils (8) 1,10 mm und der Durchmesser des Katheterlumens (7) 1,20 mm betragen.

12. Vorrichtung nach einem der Ansprüche 1 bis 11,
**dadurch gekennzeichnet, dass** der Außendurchmesser des Katheters (5) 1,50 mm beträgt.

13. Vorrichtung nach einem der Ansprüche 1 bis 12,
**dadurch gekennzeichnet, dass** die Katheterlänge etwa 25,0 mm beträgt.

14. Vorrichtung nach einem der Ansprüche 1 bis 13,
**dadurch gekennzeichnet, dass** der Mandrin (1) und der Katheter (5) durch eine Schraubenverbindung (14, 15) miteinander zu verbinden sind.

15. Vorrichtung nach Anspruch 14,
**dadurch gekennzeichnet, dass** die Schraubenverbindung (14, 15) zwischen dem Anschlussstück (10) am Katheter (5) und dem Betätigungsteil (11) am Mandrin (1) gebildet ist.

16. Vorrichtung nach Anspruch 14 oder 15,
**dadurch gekennzeichnet, dass** am Katheter (1) ein Außengewinde (14) und am Mandrin (1) ein Innengewinde (15) vorgesehen ist.

## Claims

1. Device for expanding organ or body orifices, **characterized by**
a solid and rigid mandrin (1) having a rounded mandrin tip (2), a bar-shaped mandrin portion (3) attached to the mandrin tip (2), a conical mandrin portion (4) attached to the bar-shaped mandrin portion (3), and a guiding portion (8) having an unvarying cross section an being attached to the conical mandrin portion (4);
a catheter (5) having a conical distal catheter end (6) with a cone angle (α) which is at most equal to that of the conical mandrin portion (4), and a catheter lumen (7) adapted to the cross section of the guiding portion (8); and
a stopper (9) being operative between the mandrin (1) and the catheter (5) if a dilator portion (12) of the mandrin (1), consisting of the mandrin tip, the bar-shaped mandrin portion (3) and the conical mandrin portion (4), is protruding from the distal catheter end (6).

2. Device according to claim 1,
**characterized in that** the distal conical catheter end (6) is directly attached to the larger cone end of the conical mandrin portion (4).

3. Device according to claim 1 or 2,
**characterized in that** the proximal mandrin end is fixed to an actuation part (11) and the proximal catheter end is fixed to a connector (10), wherein the stopper (9) is being formed between the actuation part (11) and the connector (10).

4. Device according to claim 3,
**characterized in that** a insertion funnel (13) comprising the proximal end of the catheter lumen (7) is provided at the connector (10).

5. Device according to claim 1,
**characterized in that** the bar-shaped mandrin portion (3) has a diameter of 0,30 mm.

6. Device according to claim 1 or 5,
**characterized in that** the bar-shaped mandrin portion (3) has a length in the range of 2,0 mm to 4,0 mm.

7. Device according to claim 1,
**characterized in that** the conical mandrin portion (4) has a length of 2,0 mm.

8. Device according to claim 1,
**characterized in that** the cone angle (α) of the conical mandrin portion is more than 22° and less than 23°.

9. Device according to claim 1,
**characterized in that** the conical portion of the distal catheter end (6) has a length of approximately 1,0 mm.

10. Device according to claim 1,
**characterized in that** the cross sections of the catheter lumen (7) and the guiding portion (8) of the mandrin (1) are formed in a circular-cylindrical shape.

11. Device according to claim 1 or 10,
**characterized in that** the diameter of the guiding portion (8) is 1,10 mm and the diameter of the catheter lumen (7) is 1,20 mm.

12. Device according to any one of the claims 1 or 11,
**characterized in that** the outer diameter of the catheter (5) is 1,50 mm.

13. Device according to any one of the claims 1 or 12,
**characterized in that** the catheter length is approximately 25,0 mm.

14. Device according to any one of the claims 1 or 13,
**characterized in that** the mandrin (1) and the catheter (5) are to be connected to each other by means of a bolted connection (14, 15).

15. Device according to claim 14,
**characterized in that** the bolted connection (14, 15) is being formed between the connector (10) at the catheter (5) and the actuating part (11) at the mandrin (1).

16. Device according to claim 14 or 15,
**characterized in that** an external thread (14) is provided at the catheter (5) and an internal thread (15) is provided at the mandrin (1).

## Revendications

1. Dispositif destiné à élargir des ouvertures dans un organe ou un organisme, **caractérisé par**
- un fil guide (1) massif et rigide avec une pointe de fil guide (2) arrondie, avec une partie de fil guide (3) en forme de barre, qui se raccorde à la pointe de fil guide (2), avec une partie de fil guide (4) conique, qui se raccorde à la partie de fil guide (3) en forme de barre, et avec une partie de guidage (8) de section transversale constante, qui se raccorde à la partie de fil guide conique (4) ;
- un cathéter (5) avec une extrémité de cathéter (6) distale, conique, qui présente au maximum le même angle de cône (α) que la partie de fil guide conique (4), et avec un lumen de cathéter (7) adapté à la section transversale de la partie de guidage (8) ; et
- une butée (9), active entre le fil guide (1) et le cathéter (5) pour la partie de dilatateur (12) du fil guide (1), qui dépasse de l'extrémité de cathéter (6) distale et est formée de la pointe du fil guide, de la partie de fil guide (3) en forme de barre et de la partie de fil guide (4) conique.

2. Dispositif suivant la revendication 1, **caractérisé en ce que** l'extrémité de cathéter (6) distale conique se raccorde directement à l'extrémité de cône la plus grande de la partie de fil guide (4) conique.

3. Dispositif suivant l'une des revendications 1 et 2, **caractérisé en ce que** l'extrémité de fil guide proximale est fixée sur un élément d'actionnement (11) et l'extrémité de cathéter proximale est fixée sur un raccord (10), la butée (9) étant formée entre l'élément d'actionnement (11) et le raccord (10).

4. Dispositif suivant la revendication 3, **caractérisé en ce qu'**un entonnoir d'insertion (13), qui entoure l'extrémité proximale du lumen de cathéter (7), est prévu sur le raccord (10).

5. Dispositif suivant la revendication 1, **caractérisé en ce que** la partie de fil guide (3) en forme de barre présente un diamètre de 0,30 mm.

6. Dispositif suivant l'une des revendications 1 et 5, **caractérisé en ce que** la partie de fil guide (3) en forme de barre présente une longueur de 2,0 mm à 4,0 mm.

7. Dispositif suivant la revendication 1, **caractérisé en ce que** la partie de fil guide (4) conique présente une longueur de 2,0 mm.

8. Dispositif suivant la revendication 1, **caractérisé en ce que** l'angle de cône (α) de la partie de fil guide conique est égal à au moins 22° et au maximum à 23°.

9. Dispositif suivant la revendication 1, **caractérisé en ce que** la partie conique de l'extrémité de cathéter (6) distale a une longueur d'environ 1,0 mm.

10. Dispositif suivant la revendication 1, **caractérisé en ce que** les sections transversales du lumen de cathéter (7) et de la partie de guidage (8) du fil guide (1) sont cylindriques circulaires.

11. Dispositif suivant l'une des revendications 1 et 10, **caractérisé en ce que** le diamètre de la partie de guidage (8) est de 1,10 mm et le diamètre du lumen de cathéter (7) est de 1,20 mm.

12. Dispositif suivant l'une des revendications 1 à 11, **caractérisé en ce que** le diamètre extérieur du cathéter (5) est de 1, 50 mm.

13. Dispositif suivant l'une des revendications 1 à 12, **caractérisé en ce que** la longueur du cathéter est d'environ 25,0 mm.

14. Dispositif suivant l'une des revendications 1 à 13, **caractérisé en ce que** le fil guide (1) et le cathéter (5) sont assemblés entre eux par une liaison par vissage (14, 15) .

15. Dispositif suivant la revendication 14, **caractérisé en ce que** la liaison par vissage (14, 15) est formée entre le raccord (10) sur le cathéter (5) et l'élément d'actionnement (11) sur le fil guide (1).

16. Dispositif suivant l'une des revendications 14 et 15, **caractérisé en ce qu'**un filetage extérieur (14) est prévu sur le cathéter (1) et un filetage intérieur (15) est prévu sur le fil guide (1).
